# EUROPEAN PATENT SPECIFICATION

(11) **EP 3 525 750 B1**
(45) Date of publication and mention of the grant of the patent: **09.07.2025**
(21) Application number: 17783820.8
(22) Date of filing: 09.10.2017
(51) Int. Cl.: A61K 8/29, A61K 8/60, A61Q 17/04

(54) **COSMETIC COMPOSITIONS**
KOSMETISCHE ZUSAMMENSETZUNGEN
COMPOSITIONS COSMÉTIQUES

(30) Priority: 11.10.2016 EP 16193201
(43) Date of publication of application: 21.08.2019
(73) Proprietor: DSM IP Assets B.V., 6221 BE Maastricht (NL)
(72) Inventor: DESHAYES, Cyrille, 4303 Kaiseraugst (CH); HOELLER, Ulrich, 4303 Kaiseraugst (CH); JANSSEN, Anne, 4303 Kaiseraugst (CH)
(74) Representative: dsm-firmenich IP
(86) International application number: PCT/EP2017/075598
(87) International publication number: WO 2018/069207

(56) References cited:
- EP-A2- 1 068 866
- WO-A1-2009/003934
- WO-A1-2015/044306
- WO-A2-2009/063392
- WO-A2-2009/103651
- US-A1- 2009 180 976
- US-A1- 2013 266 621
- ANONYMOUS: "DECYL GLUCOSIDE | 68515-73-1", 9 October 2020 (2020-10-09), XP055738452, Retrieved from the Internet <URL:https://www.chemicalbook.com/ChemicalProductProperty_EN_CB4965996.htm> [retrieved on 20201009]
- ANONYMOUS: "Decyl glucoside", 16 April 2021 (2021-04-16), XP093232404, Retrieved from the Internet <URL:https://www.chemicalbook.com/ChemicalProductProperty_DE_CB0965701.htm>
- ANONYMOUS: "Decyl Glucoside", 19 April 2021 (2021-04-19), XP093232410, Retrieved from the Internet <URL:https://www.personalcarecouncil.org/resizrces/inci/>

## Description

The present invention relates to an aqueous dispersions consisting of water, a nano-sized, organic, insoluble UV filter and a C₈₋₁₀ alkyl poly-glucoside as well as to cosmetic compositions comprising said dispersion in combination with at least one inorganic micropigment, characterized in that said cosmetic compositions are substantially free of any C₁₂₋₁₆ alkyl poly-glucoside.

WO 2009/003934 A1 discloses methods of preparing compositions comprising a micronised insoluble organic UV absorber by grinding the absorber in the presence of an alkyl polyglucoside.

Tinosorb^{®} M is an organic UV filter which uses microfine particle technology and acts as both a micropigment and an UV absorber. It is sold as a dispersion of methylene bis-benzotriazolyl tetramethylbutylphenol in water, wherein the particles are stabilized by a decyl glucoside consisting of C₈ to C₁₆ alkyl poly-glucosides. Tinosorb^{®} M is not only widely used in sunscreen but also in day care as well as in skin lightening products. A drawback of Tinosorb^{®} M is, however, that the combination thereof with inorganic micropigments in a cosmetic composition often leads to an unwanted 'cottage-cheese' like appearance of said composition which is highly unwanted by the cosmetic industry.

Thus, there is an ongoing need for a stable aqueous dispersion of nano-sized methylene bis-benzotriazolyl tetramethylbutylphenol which can readily be incorporated into cosmetic composition in combination with an inorganic micropigment and which overcomes the above mentioned drawback.

Surprisingly it has been found, that an aqueous dispersion of nano-sized methylene bis-benzotriazolyl tetramethylbutylphenol, wherein the particles are stabilized by a C₈-₁₀ alkyl poly-glucoside allows the formulation of aesthetically appealing compositions which comprise, next to the dispersion at least one inorganic micropigment.

Thus, in a first embodiment, the invention relates to an aqueous dispersions (I) consisting essentially of
(i) 30-70 wt.-%, preferably 40-60 wt.-%, based on the total weight of the aqueous dispersion, of a nano-sized, organic, insoluble UV-absorber,
(ii) 2 to 15 wt.-%, preferably 5 to 10 wt.-%, based on the total weight of the aqueous dispersion, of a C₈₋₁₀ alkyl poly-glucoside consisting essentially of caprylyl (C₈) and capryl (C₁₀) poly-glucoside,
(iii) 0 to 3 wt.-%, preferably 0.1 to 1 wt.-%, based on the total weight of the aqueous dispersion of at least one additive, and
(iv) 25 to 60 wt.-%, preferably 30 to 45 wt.-%, based on the total weight of the aqueous dispersion, of water.

In another embodiment, the present invention relates to a cosmetic composition comprising at least one inorganic micropigment and an aqueous dispersion (I), characterized in that the cosmetic composition is substantially free of any C₁₂₋₁₆ alkyl poly-glucoside.

Furthermore, the present invention relates to the use of a C₈₋₁₀alkyl poly-glucoside for stabilizing a mixture of nano-sized, organic, insoluble UV-absorber and inorganic micropigments in a cosmetic composition and in the absence of any C₁₂₋₁₆ alkyl poly-glucosides.

Additionally, the present invention relates to a method for stabilizing a mixture of nano-sized, organic, insoluble UV-absorber and inorganic micropigments in a cosmetic composition, wherein said method comprises the addition of a C₈₋₁₀alkyl poly-glucoside and the omission of the addition of any C₁₂₋₁₆ alkyl poly-glucosides to said composition.

In a further embodiment, the invention relates to a method for the preparation of a cosmetic composition, said method encompassing the step of admixing at least one inorganic micropigment and a dispersion (I) with a cosmetically acceptable carrier, with the proviso that no C₁₂₋₁₆ alkyl poly-glucoside is admixed and/ or present in said composition.

The term 'consisting essentially of' as used according to the present invention means that the amounts of the ingredients (i) to (iv) sum up to 100 wt.-%. It is, however, not excluded that small amount of impurities or additives may be present which are, for example, introduced via the respective raw materials of the ingredients (i) to (iv).

The term "substantially (i.e. essentially) free of any C₁₂₋₁₆ alkyl poly-glucoside" as defined herein means that the compositions of the present invention contain no appreciable amount of C₁₂₋₁₆ alkyl poly-glucosides, in particular no amounts which lead to the adverse effect in combination with the inorganic micropigment, i.e. no more than 0.1 wt.-%, preferably no more than 0.05 wt.-%, most preferably no more than 0.01% such as in particular no more than 0.005 wt.-%, based on the total weight of the cosmetic compositions.

The term 'insoluble' as used herein refers to an UV absorbers which exhibits a solubility at RT (i.e. ~ 22°C) in common cosmetic oils such as e.g. C₁₂₋₁₅ alkyl benzoate, propyleneglycol, mineral oil but also in water of less than 0.01 wt.-%, preferably of less than 0.05 wt.-%, most preferably of less than 0.03 wt.-%.

The term 'additive' as used herein refers to additives commonly used in the preparation of aqueous dispersions of nano-sized, organic, insoluble UV-absorber such as in particular to wetting agents, anti-foaming agents and thickeners as well as mixtures thereof.

Particularly suitable wetting agents according to the present invention encompass (poly)propyleneglycol and/ or butylene glycol as well as mixtures thereof. Most preferably in all embodiments of the present invention the wetting agent is propyleneglycol. Such wetting agent(s) are preferably present in the dispersion (I) in an amount (total) selected in the range of 0.1 to 1 wt.-%, more preferably in an amount of 0.2 to 0.6 wt.-%, based on the total weight of the dispersion.

Particularly suitable anti-foaming agents according to the present invention encompass silicone oils such as in particular polydimethylsiloxanes and/ or silicon anti-foam agents such as in particular anhydrous dispersions of pyrogenic or hydrophobized silica in silicone oils such as most in particular simethicone. Most preferably in all embodiments of the present invention the anti-foaming agent is simethicone. Such anti-foaming agent(s) are preferably present in the dispersion (I) in an amount (total) selected in the range of 0 to 1 wt.-%, more preferably in an amount of 0.01 to 0.2 wt.-%, based on the total weight of the dispersion.

Particularly suitable thickeners according to the present invention encompass xanthan gum, gellan gum and/ or carboxymethylcellulose. Most preferably in all embodiments of the present invention the thickener is xanthan gum or gellan gum. Such thickener(s) are preferably present in the dispersion (I) in an amount (total) selected in the range of 0.1 to 1 wt.-%, more preferably in an amount of 0.1 to 0.5 wt.-%, based on the total weight of the dispersion.

It is well understood, that one or more additives may be present in the aqueous dispersion (I). In a particular advantageous embodiment, at least one thickener and at least on wetting agent is present in the aqueous dispersions (I). Most preferably, the aqueous dispersion (I) contains as additives propyleneglycol and one thickener selected from xanthan gum or gellan gum. Preferably, in all embodiments of the present invention, the insoluble organic UV absorbers is selected from the group consisting of 2,2'-methylene-bis-(6(2H-benzotriazol-2-yl)-4-(1,1,3,3-tetramethylbutyl)-phenol [INCI: methylene bis-benzotriazolyl tetramethylbutylphenol, CAS 103597-45-1] and 2,4,6-tris(biphenyl-4-yl)-1,3,5-triazin (INCI: Tris-Biphenyl Triazine, CAS 31274-51-8). Most preferably in all embodiments of the present invention the insoluble organic UV absorbers is methylene bis-benzotriazolyl tetramethylbutylphenol.

The nano-sized insoluble organic UV absorber has a mean particle size distribution Dᵥ50 determined by light scattering (i.e. by Photon Correlation Spectroscopy (PCS)) of less than 200 nm, more preferably in the range of 50 to 150 nm, most preferably in the range of 75 to 125 nm, such as in particular in the range of 80 to 110 nm. In a particular advantageous embodiment, the nano-sized insoluble organic UV absorber exhibits a Dᵥ10 in the range of 50 to 80 nm, a Dᵥ50 in the range of 75 to 125 nm and a Dᵥ90 in the range of 140 to 180 nm, and even more preferably a Dᵥ10 in the range of 55 to 75 nm, a Dᵥ50 in the range of 80 to 110 nm and a Dᵥ90 in the range of 150 to 175 nm. The particle size as given herein is generally determined in a suspension of the nano-sized insoluble organic UV absorber in water such as ultrapure water (Mili-Q purified), preferably at a concentration level of 3 mg/ml using a Beckman Coulter Delsa Nano S.

The term 'alkyl poly-glucoside (APG)' refers to a class of non-ionic surfactants having the generic formula CₙH₂₊ₙ O(C₆H₁₀O₅)ₓH, in which n is an integer selected in the range of 2 to 22 and x refers to the mean polymerization level of the glucoside moiety (i.e. to the respective mono-, di-, tri-, oligo-, and poly-glucosides as well as mixtures thereof). These APG's are widely used in household and industrial applications. They are generally derived from renewable raw materials such as glucose derived from corn and plant derived fatty alcohols. These alkyl poly-glucosides generally exhibit a mean polymerisation level of the glucoside moiety ranging from 1 to 1.7, preferably from 1.2 to 1.6 such as from 1.4 to 1.6.

Further advantageous mean polymerisation level of the glucoside moiety range from 1.1 to 1.6, such as from 1.1 to 1.4 or from 1.1 to 1.3. Additional advantageous mean polymerisation level of the glucoside moiety range from 1.2 to 1.7, respectively from 1.4 to 1.6. C₈₋₁₀ alkyl poly-glucoside according to the invention consists essentially of caprylyl (C₈) and capryl (C₁₆) poly-glucosides. Preferably such caprylyl (C₈) and capryl (C₁₀) poly-glucosides furthermore exhibit a ratio (%/%, wherein all % are area-% determined by HPLC-MS) of caprylyl (C₈) mono-glucoside to capryl (C₁₀) mono-glucoside in the range of 3:1 to 1:3, preferably in the range of about 2:1 to 1:2, most preferably in the range of 1.5:1 to 1:1.5. It is understood, that such alkyl poly-glucosides are basically free of any (i.e. contain no) higher (i.e. C₁₄₋₁₆) alkyl polyglucosides.

Furthermore, the C₈₋₁₀ alkyl poly-glucosides according to the invention consisting essentially of caprylyl (C₈) and capryl (C₁₀) poly-glucosides contain advantageously at least 60%, preferably at least 65%, most preferably at least 70% of the respective mono-glucosides as e.g. determined by HPLC-MS.

The C₈₋₁₀ alkyl poly-glucoside according to the present invention are substantially (essentially) free of any C₉ alkyl poly-glucosides, i.e. contain essentially no C₉ alkyl poly-glucosides. This means that the amount of any C₉ alkyl poly-glucosides in the C₈₋₁₀ alkyl poly-glucoside is less than 0.1 wt.-%, preferably less than 0.05 wt.-%, most preferably less than 0.01% such as in particular less than 0.005 wt.-%, based on the total weight of the C₈₋₁₀ alkyl poly-glucoside.

A particularly advantageous C₈₋₁₀ alkyl poly-glucoside according to the present invention is made from glucose derived from corn and C₈ and C₁₀ fatty alcohols derived from coconut and palm kernel oils, which is e.g. sold as an aqueous dispersion under the tradename Green APG 0810 by Shanghai Fine Chemical.

Particular advantageous aqueous dispersions (I) according to the present invention are aqueous dispersions (II) consisting of
(i) 45-55 wt.-%, based on the total weight of the aqueous dispersion, of nano-sized, methylene bis-benzotriazolyl tetramethylbutylphenol having a Dᵥ50 determined by light scattering in the range of 50-150 nm,
(ii) 5 to 10 wt.-%, based on the total weight of the aqueous dispersion, of C₈₋₁₀ alkyl poly-glucoside,
(iii)
   a) 0.1 to 0.7 wt.-& of propylene glycol,
   b) 0.1 to 0.7 wt.-% of gellan gum or xanthan, and
(iv) 35 to 45 wt.-%, based on the total weight of the aqueous dispersion, of water.
(It is well understood, that all the definitions and preferences as given herein also apply to the dispersions (II)).

The aqueous dispersions according to the present invention are advantageously incorporated into the cosmetic compositions according to the present invention in an amount 0.1 to 20 wt.-%, preferably in an amount of 0.5 to 10 wt.-% most preferably in an amount of 1 to 5 wt.-%, based on the total weight of the cosmetic composition.

The inorganic micropigments are advantageously incorporated into the cosmetic compositions according to the present invention in a total amount selected in the range of 0.1 to 40 wt.-%, preferably in an amount selected in the range of 1 to 30 wt.-%, based on the total weight of the cosmetic composition.

Particularly suitable inorganic micropigments in all embodiments of the present invention are metal powders, metal oxides or metal hydroxides conventionally used in cosmetic applications either as inorganic UV filter or as colouring agent. Exemplary inorganic micropigments according to the present invention encompass magnesium oxide, magnesium hydroxide, calcium oxide, calcium hydroxides, aluminum oxide, aluminum hydroxide, iron oxides (α-Fe₂O₃, γ-Fe₂O₃, Fe₃O₄, FeO), red iron oxide, yellow iron oxide, black iron oxide, iron hydroxides, titanium (di)oxides, zirconium oxides, chromium oxides, chromium hydroxides, manganese oxides, cobalt oxides, cerium oxides, nickel oxides and zinc oxides as well as composite oxides and composite hydroxides such as iron titanate, cobalt titanate and cobalt aluminate.

The inorganic micropigments according to the present invention may optionally be surface treated to, for example, make the particles more hydrophobic or more dispersible in a vehicle.

Particularly preferred inorganic micropigments according to the present invention are selected from the group consisting of titanium dioxides, zinc oxides and iron oxides, most preferably from titanium dioxide and iron oxide, as well as mixtures thereof.

In one particular advantageous embodiment the inorganic micropigment is an inorganic UV filter having a particle size which is principally useful for incorporation into a sunscreen composition such as in particular a titanium dioxide or zinc oxide UV filter.

These inorganic UV filters are preferably used in an amount (total) selected in the range of 0.1 to 20 wt.-%, preferably in the range of 0.5 to 10 wt.-%, more preferably in the range of 1 to 10 wt.-%, based on the total weight of the cosmetic composition.

Preferably, in all embodiments of the present invention, a titanium dioxide UV filter having an average primary particle size of about 2 nm to 100 nm, preferably of about 5 to 50 nm and a secondary particle size of about 0.05 to 50 µm, preferably of about 0.1 to1 µm is used.

The crystalline form of the titanium dioxide UV filter may be of any crystal or amorphous type. For example, titanium dioxide may be any type of amorphous, rutil, anastase, brookite or a mixture thereof.

In a preferred embodiment, the titanium dioxide UV filter used according to the present invention is coated with at least one coating such as in particular with aluminium hydroxide, a polyol, silica, a silicon oil such as methicone or dimethicone, or an alkyl silane. Such coatings are well known in the art. Commercially available single coated titanium dioxides suitable according to the invention are e.g. available as Uvinul^{®} TiO₂ (INCI: trimethoxycaprylylsilane and titanium dioxide ex BASF) or Eusolex^{®} T-Avo (INCI: Titanium dioxide, Silica ex Merck).

In a more particular embodiment of the invention, the titanium dioxide UV filter, however, is a double coated titanium dioxide as this leads to even better results. Such double coated titanium dioxide preferably have an inner coating selected from inorganic silica or aluminium hydroxide and an outer organic coating (referred to as double coated titanium dioxide). Preferably the outer organic coating is selected from silicone oils (e.g. simethicones, methicones, dimethicones, polysilicone-15), alkyl silanes, olefinic acids such as in particular stearic acid, polyols such as in particular glycerol or organophosphonic acids such as in particular cetyl phosphate.

In such double coated titanium dioxide the inner coating preferably consists of minimum 0.5 wt.-%, more preferably of 0.5-50 wt.-%, most preferably of 1-20 wt.-%, based on the weight of the non-coated titanium dioxide.

The outer coating layer preferably consists of minimum 0.25 wt.-%, preferably of 0.5-50 wt.-%, most preferably of 0.5-10 wt.-% of organic coating, based on the weight of the non-coated titanium dioxide.

Such double coated titanium dioxides nanoparticles can be prepared according to the state of the art or are commercially available as PARSOL^{®} TX (INCI: Titanium Dioxide, Silica, Dimethicone ex DSM Nutritional Products) or as UV-Titan X195 (coated with silica and treated with a silicone oil (i.e. methicone) ex Merck) or Tayca MT-100TV (Titanium Dioxide (and) Aluminum Hydroxide (and) Stearic Acid).

Other usual organic coatings can additionally be present in order to yield multiple coated (such as e.g. triple coated) titanium dioxide. The other coatings can be applied before, after or together with the second outer coating. Other additional coatings which can be used comprise organic coatings such as stearic acid, silicones (silane derivatives such as triethoxycaprylylsilane or siloxane derivatives such as methicone, dimethicone, simethicone).

In all embodiments of the present invention the titanium dioxide UV filter is most preferably a double coated titanium dioxide having an inner inorganic silica coating wherein the outer coating consists of simethicone, methicone, dimethicone (also known as polydimethylsiloxane), polysilicone-15, stearic acid, glycerol and mixtures thereof, in particular of methicone, dimethicone, stearic acid or mixtures thereof. Most preferably, the outer coating consists of methicone or dimethicone, in particular of dimethicone. The most preferred the titanium dioxide UV filter according to the invention are UV-Titan X195 by Huntsman and/or PARSOL^{®} TX by DSM Nutritional products which are titanium dioxide grades coated with silica (inner coating) and treated with a silicone oil such as in particular methicone (UV-Titan X195) or dimethicone (PARSOL^{®} TX) as outer coating. Most in particular PARSOL^{®} TX by DSM Nutritional products is used as titanium dioxide UV filter in the compositions according to the invention.

In another advantageous embodiment, the inorganic micropigment is a coloring agent conventionally used in decorative cosmetics such as make-up and/ or foundation compositions. Particularly suitable inorganic coloring agents according to the present invention are titanium dioxide, zirconium or cerium oxides, zinc, iron (black, yellow or red) or chromium oxides, manganese violet, ultramarine blue, chromium hydrate and ferric blue, or metal powders, such as aluminium powder or copper powder.

If present, the amount (total) of these inorganic coloring agent(s) is preferably selected in the range of 1 wt.-% to 40 wt.-%, preferably in the range of 2 wt.-% to 30 wt.-%, more preferably in the range of 5 wt.-% to 15 wt.-%, based on the total weight of the cosmetic composition.

The crystalline form of the iron and titanium dioxide coloring agent may be of any crystal or amorphous type suitable for that purpose. For example, titanium dioxide may be any type of amorphous, rutil, anastase, brookite or a mixture thereof. The particle shape of the iron oxide coloring agent may be of any acicular, spheroidal or cubic shape, as well as mixtures thereof.

Particularly preferred inorganic coloring agent according to the present invention are selected from the group consisting of iron oxide and titanium dioxide having a particle size ranging from about 0.001 to 150 µm, preferably from about 0.002 to 100 µm, more preferably from about 0.02 to 50 µm. Such inorganic coloring agents are well known to a person skilled in the art and e.g. commercially available under the tradename UNIPURE at Sensient.

In a preferred embodiment, the iron oxide and titanium dioxide coloring agents used according to the present invention are surface treated with an organic coating such as with an alkylsilane e.g. triethoxycaprylylsilane, with a silicone oil e.g. dimethicone or methicone, with an organo titanate, and/or with natural surface treatments e.g. polyhydroxystearic acid, stearoyl glutamic acid hydrogenated lecithin, jojoba esters and sodium glycerophosphate. Such coated inorganic coloring agents are well known to a person skilled in the art and e.g. commercially available under the tradename UNIPURE at Sensient, or from the product portfolio for coloring agents at KOBO, Merck.

Particularly suitable inorganic coloring agents for foundation and/ or make-up compositions according to the present invention include optionally surface treated titanium dioxides (rutile or anatase) listed in the Color Index under reference CI 77891 such as UNIPURE LC 981 AS-EM from Sensient. Further suitable inorganic coloring agents for foundation and/ or make-up compositions according to the present invention include black, yellow, red and brown iron oxides, optionally surface treated, listed in the Color Index under references CI 77499, 77492 and 77491 such as Unipure RED LC381 from Sensient.

The cosmetic compositions according to the invention are intended for topical application, which is to be understood as the external application to keratinous substances, such as in particular the skin.

As the cosmetic compositions according to the invention are intended for topical application, they comprise a physiologically acceptable medium, that is to say a medium compatible with keratinous substances, such as the skin, mucous membranes, and keratinous fibers. In particular the physiologically acceptable medium is a cosmetically acceptable carrier.

The term 'cosmetically acceptable carrier' as used herein refers to a physiologically acceptable medium which is compatible with keratinous substances. Suitable carriers are well known in the art and are selected based on the end-use application. Preferably, the carriers of the present invention are suitable for application to skin (e.g. in the form of creams, milks, lotions, masks, serums, hydrodispersions, foundations, creamgels, or gels etc.). Such carriers are well-known to one of ordinary skill in the art and can include one or more compatible liquid or solid filler diluent, excipient, adjuvant, additive or vehicle which are suitable for application to skin.

Examples of cosmetic excipients, diluents, adjuvants, additives as well as active ingredients commonly used in the skin care industry which are suitable for use in the cosmetic compositions of the present invention are for example described in the International Cosmetic Ingredient Dictionary & Handbook by Personal Care Product Council (http://www.personalcarecouncil.org/), accessible by the online INFO BASE (http://online.personalcarecouncil.org/jsp/Home.jsp), without being limited thereto.

Particularly suitable excipients, diluents, adjuvants additives for the compositions according to the present invention are cosmetic oils such as C12-15 alkyl benzoate, cetyl alcohol, cetearyl alcohol, capric/caprylic triglycerides, diisopropylsebacate, preservatives such as phenoxyethanol and ethlyhexylglycerin (Euxyl PE 9010 from Shülke & Mayr), parabens (Euxyl K 300 form Schülke&Mayr); thickening agents for the aqueous phase such as polysaccharide such as e.g. Xanthan Gum (Keltrol CGT from Kelco); biopolymers such as e.g. cellulose gum (Tylose CG 200 from SE Tylose); mineral thickeners such as e.g. magnesium aluminium silicate (Veegum from Vanderbilt), synthetic polymers such as e.g. carbomer (Carbopol 980 from Lubrizol), UV filters, fragrances as well as humectants such as e.g. glycerin and propylene glycol.

In accordance with the present invention, the compositions according to the invention may also comprise further cosmetically active ingredients conventionally used in cosmetic compositions. Exemplary active ingredients encompass skin lightening agents, UV filters, agents for the treatment of hyperpigmentation, agents for the prevention or reduction of inflammation, firming, moisturizing, soothing and/ or energizing agents as well as agents to improve elasticity and skin barrier.

The necessary amounts of the active ingredients as well as the excipients, diluents, adjuvants, additives etc. can, based on the desired product form and application, easily be determined by the skilled person. The additional ingredients can either be added to the oily phase, the aqueous phase or separately as deemed appropriate.

The cosmetically active ingredients useful herein can in some instances provide more than one benefit or operate via more than one mode of action.

Of course, one skilled in this art will take care to select the above mentioned optional additional ingredients, adjuvants, diluents and additives and/or their amounts such that the advantageous properties intrinsically associated with the combination in accordance with the invention are not, or not substantially, detrimentally affected by the envisaged addition or additions.

The cosmetic compositions according to the present invention are typically prepared by admixing the aqueous dispersion according to the present invention and the inorganic micropigment with suitable excipients, diluents, adjuvants and/ or additives. If desired, active ingredients can additionally be added to the cosmetic compositions according to the present invention.

In a particularly preferred embodiment, the cosmetic compositions according to the present invention are sunscreen compositions for the protection of the skin against harmful UV-radiation or make-up and/ or foundation compositions for the provision of a uniform "base" skin color.

The compositions according to the present invention, in particular the sunscreen compositions, preferably comprise at least one further organic UV-filter substance (light screening agents) which is active in the UV-A and/or UV-B regions (absorbers), such UV-filter substances being watersoluble, fat-soluble or insoluble in commonly used cosmetic solvents.

Particularly advantageous UVA, UVB and/ or broadspectrum UV-filter substances to be incorporated into the cosmetic compositions according to the present invention are dibenzoylmethane derivatives such as e.g. butyl methoxydibenzoylmethane (PARSOL^{®} 1789); acrylates such as e.g. octocrylene (PARSOL^{®} 340); camphor derivatives such as e.g. 4-methyl benzylidene camphor (PARSOL^{®} 5000) or terephthalylidene dicamphor sulfonic acid (Mexoryl^{®} SX); cinnamate derivatives such as e.g. ethylhexyl methoxycinnamate (PARSOL^{®} MCX) or isoamyl methoxycinnamate; p aminobenzoic acid derivatives such as e.g. p aminobenzoic acid or 2-ethylhexyl p-dimethylaminobenzoate; benzophenones such as e.g. benzophenone-3, benzophenone-4, 2,2',4,4'-tetrahydroxy-benzophenone or 2,2'-dihydroxy-4,4'-dimethoxybenzophenone; esters of benzalmalonic acid such as e.g. di-(2-ethylhexyl) 4-methoxybenzalmalonate; organosiloxane compounds carrying chromophore groups such as e.g. polysilicone-15 (PARSOL^{®} SLX) or drometrizole trisiloxane (Mexoryl^{®} XL); imidazole derivatives such as e.g. 2-phenyl benzimidazole sulfonic acid and salts thereof such as e.g. its sodium- or potassium salts (PARSOL^{®} HS); salicylate derivatives such as e.g. ethylhexyl salicylate (PARSOL^{®} EHS, Neo Heliopan^{®} OS), isooctyl salicylate or homosalate (PARSOL^{®} HMS, Neo Heliopan^{®} HMS); triazine derivatives such as e.g. ethylhexyl triazone (Uvinul^{®} T-150), diethylhexyl butamido triazone (Uvasorb^{®} HEB), bis-ethylhexyloxyphenol methoxyphenyl triazine (Tinosorb^{®} S) or Tris-Biphenyl Triazine (2,4,6-Tris(biphenyl-4-yl)-1,3,5-triazin, Tinosorb^{®} A2B); encapsulated UV-filters such as e.g. encapsulated ethylhexyl methoxycinnamate (Eusolex^{®} UV-pearls); amino substituted hydroxybenzophenones such as e.g. diethylamino hydroxybenzoyl hexyl benzoate (Aminobenzophenon, Uvinul^{®} A Plus); benzoxazol-derivatives such as e.g. 2,4-bis-[5-1 (dimethylpropyl)benzoxazol-2-yl-(4-phenyl)-imino]-6-(2-ethylhexyl)-imino-1,3,5-triazin (Uvasorb^{®} K2A); phenylene-1,4-bis-benzimidazolsulfonic acids or salts thereof such as e.g. disodium phenyl dibenzimidazole tetrasulfonate (2,2-(1,4-phenylene)bis-(1H-benzimidazol-4,6-disulfonic acid, Neoheliopan^{®} AP); 1,1' (1,4-piperazinediyl)bis[1-[4-(diethylamino)-2-hydroxybenzoyl]phenyl]-methanone (CAS No. 919803-06-6); as well as Bis(butylbenzoate) diaminotriazine aminopropyltrisiloxane (CAS No. 207562-42-3).

Preferred UVB-filter substances to be incorporated into the cosmetic compositions according to the invention encompass polysilicone-15, phenylbenzimidazol sulfonic acid, octocrylene, ethylhexyl methoxycinnamate, ethyl hexylsalicylate, tris-biphenyl triazine and/ or homosalate.

Preferred broadband UV-filter substances to be incorporated into the cosmetic compositions according to the invention encompass unsymmetrical s triazine derivatives such as in particular bis-ethylhexyloxyphenol methoxyphenyl triazine, and/ or certain benzophenones such as e.g. 2-hydroxy-4-methoxy-benzophenon.

Preferred UVA-filter substances to be incorporated into the cosmetic compositions according to the invention encompass butyl methoxydibenzoylmethane, diethylamino hydroxybenzoyl hexyl benzoate, 2,4-bis-[5-1(dimethylpropyl)benzoxazol-2-yl-(4-phenyl)-imino]-6-(2-ethylhexyl)-imino-1,3,5-triazine and/ or disodium phenyl dibenzimidazole tetrasulfonate, in particular butyl methoxydibenzoylmethane and/ or diethylamino hydroxybenzoyl hexyl benzoate.

If the topical sunscreen emulsions comprise butyl methoxydibenzoylmethane, then they advantageously contain in addition at least one suitable photostabilizer for butyl methoxydibenzoylmethane. Besides specific UV-filters listed above which are known to a person skilled in the art to be able to photostabilize butyl methoxydibenzoylmethane, further exemplary photostabilizers encompass Polyester 8 (Polycrylene^{®}); Methoxycrylene (Solastay); diethylhexyl syringylidene malonate (Oxynex ST liquid); diethylhexyl naphthalate (Corapan TQ) as well as Benzotriazolyl Dodecyl p-Cresol (Tinogard^{®} TL) without being limited thereto. An overview on such photostabilizers is e.g. given in 'SPF Boosters & Photostability of Ultraviolet Filters', HAPPI, October 2007, p. 77-83 which is included herein by reference. These photostabilizers are generally used in an amount of 0.05 to 10 wt. % with respect to the total weigh of the topical sunscreen emulsion.

The total amount of the additional UV-filter substances in the compositions according to the present invention is preferably selected in the range of 0.1 to 40 wt. %, more preferably in the range of 0.2 to 20 wt. % and most preferably in the range of 0.5 to 15 wt.-%, based on the total weight of the cosmetic composition.

The compositions according to the present invention may be in the form of a suspension or dispersion in solvents or fatty substances, or alternatively in the form of an emulsion or micro emulsion (in particular of oil-in-water (O/W) or water-in-oil (W/O) type, silicone-in-water (Si/W) or water-in-silicone (W/Si) type, PIT-emulsion, multiple emulsion (e.g. oil-in-water-in oil (O/W/O) or water-in-oil-in-water (W/O/W) type), pickering emulsion, hydrogel, alcoholic gel, lipogel, one- or multiphase solution or vesicular dispersion or other usual forms, which can also be applied by pens, as masks or as sprays.

If the composition is an emulsion, such as in particular an O/W, W/O, Si/W, W/Si, O/W/O, W/O/W multiple or a pickering emulsion, then the amount of the oily phase present in such cosmetic emulsions is preferably at least 10 wt.-%, such as in the range of 10 to 60 wt.-%, preferably in the range of 15 to 50 wt.-%, most preferably in the range of 15 to 40 wt.-%, based on the total weight of the composition.

In one embodiment, the compositions according to the present invention are advantageously in the form of an oil-in-water (O/W) emulsion comprising an oily phase dispersed in an aqueous phase in the presence of an O/W emulsifier. The preparation of such O/W emulsions is well known to a person skilled in the art.

If the composition according to the invention is an O/W emulsion, then it contains advantageously at least one O/W- or Si/W-emulsifier selected from the list of, glyceryl stearate citrate, glyceryl stearate SE (self-emulsifying), stearic acid, salts of stearic acid, polyglyceryl-3-methylglycosedistearate. Further suitable emulsifiers are phosphate esters and the salts thereof such as cetyl phosphate (e.g. as Amphisol^{®} A from DSM Nutritional Products Ltd.), diethanolamine cetyl phosphate (e.g. as Amphisol^{®} DEA from DSM Nutritional Products Ltd.), potassium cetyl phosphate (e.g. as Amphisol^{®} K from DSM Nutritional Products Ltd.), sodium cetearylsulfate, sodium glyceryl oleate phosphate, hydrogenated vegetable glycerides phosphate and mixtures thereof. Further suitable emulsifiers are sorbitan oleate, sorbitan sesquioleate, sorbitan isostearate, sorbitan trioleate, cetearyl glucoside, lauryl glucoside, decyl glucoside, sodium stearoyl glutamate, sucrose polystearate and hydrated polyisobutene. Furthermore, one or more synthetic polymers may be used as an emulsifier. For example, PVP eicosene copolymer, acrylates/C₁₀₋₃₀ alkyl acrylate crosspolymer, and mixtures thereof.

The at least one O/W, respectively Si/W emulsifier is preferably used in an amount of 0.5 to 10 wt. %, in particular in the range of 0.5 to 6 wt.-%, such as more in particular in the range of 0.5 to 5 wt.-%, such as most in particular in the range of 1 to 4 wt.-%, based on the total weight of the composition.

Particular suitable O/W emulsifiers to be used in the compositions according to the invention encompass phosphate ester emulsifiers such as advantageously 8-10 alkyl ethyl phosphate, C9-15 alkyl phosphate, ceteareth-2 phosphate, ceteareth-5 phosphate, ceteth-8 phosphate, ceteth-10 phosphate, cetyl phosphate, C6-10 pareth-4 phosphate, C12-15 pareth-2 phosphate, C12-15 pareth-3 phosphate, DEA-ceteareth-2 phosphate, DEA-cetyl phosphate, DEA-oleth-3 phosphate, potassium cetyl phosphate, deceth-4 phosphate, deceth-6 phosphate and trilaureth-4 phosphate.

A particular suitable O/W emulsifier to be used in the compositions according to the invention is potassium cetyl phosphate e.g. commercially available as Amphisol^{®} K at DSM Nutritional Products Ltd Kaiseraugst.

Another particular suitable class of O/W emulsifiers are non-ionic self-emulsifying systems derived from olive oil e.g. known as (INCI Name) cetearyl olivate and sorbitan olivate (chemical composition: sorbitan ester and cetearyl ester of olive oil fatty acids) sold under the tradename OLIVEM 1000.

In one particular embodiment, the invention relates to compositions with all the definitions and preferences given herein in the form of O/W emulsions comprising an oily phase dispersed in an aqueous phase in the presence of an O/W emulsifier wherein the O/W emulsifier is potassium cetyl phosphate. The amount of oily phase in such O/W emulsions is preferably at least 10 wt.-%, more preferably in the range of 10 to 60 wt.-%, most preferably in the range of 15 to 50 wt.-%, such as in the range of 15 to 40 wt.-%.

The compositions according to the invention in general have a pH in the range of 3 to 10, preferably a pH in the range of 4 to 8 and most preferably a pH in the range of 4 to 7.5. The pH can easily be adjusted as desired with suitable acids, such as e.g. citric acid, or bases, such as sodium hydroxide (e.g. as aqueous solution), triethanolamine (TEA Care), tromethamine (Trizma Base) and Aminomethyl Propanol (AMP-Ultra PC 2000), according to standard methods in the art.

The following examples are provided to further illustrate the compositions and effects of the present invention. These examples are illustrative only and are not intended to limit the scope of the invention in any way.

### Examples

### 1. Preparation of a dispersion according to the present invention

22 kg of purified water was added into a 100l vessel at 30-35°C. Afterwards 9.8 kg Green APG 0810 were added. Then 33 kg Grandsorb UV360 having a coarse particle size Dv90 of 86 µm (measured by laser diffraction with a Malvern Mastersizer 3000, powder measurement, air pressure 0.2bar) was slowly added over a time period of 30 minutes followed by degassing the resulting suspension for 2h under gentle stirring at 65°C. The resulting suspension was then cooled down to 25-30°C. Afterwards 50 kg of the resulting suspension was milled in a LMZ 4 using yttrium-stabilized zirconium oxide grinding beads (0.3mm, 95% ZrO₂, 5% Y₂O₃ from Tosoh Ceramic, Japan) until a particle size Dᵥ50 of about 100nm (measured by light scattering with a Coulter Delsa Nano S, at an adjusted concentration of 3 mg/ ml) was obtained. After removal of the grinding beads, a suspension consisting of 161 g of propyleneglycol and 80.5 g of gellan gum was slowly added under gentle stirring at about 40°C resulting in the final product form.

### 2. Analytics

The respective methylene bis-benzotriazolyl tetramethylbutylphenol dispersion, approx. 1 mg/ml, of has been dissolved in a mixture of tetrahydrofurane / water (50/50), and was analyzed by HPLC mass spectrometry using a reversed-phase YMC Pro C4 column with a water/acetonitrile gradient with 0.1% methanesulfonic acid (5 -> 90 % acetonitrile over 15 min). Detection was performed on an Agilent 6130 single MSD operating in ES positive mode. TIC and EIC were used to determine the relative distribution of the compounds of interest.

The relative distribution of the alkyl poly-glycosides is outlined in table 1. All % are area-%.

**Table 1: Relative distribution of the respective alkyl monoglucosides**

| | ***Tinosorb*^{®} *M*** (Lot 0004694551) | ***Dispersion of example 1*** |
|---|---|---|
| C₈ mono-glucoside | 21.6 % | 49.4 % |
| C₁₀ mono-glucoside | 21.8 % | 51.6 % |
| C₁₂ mono-glucoside | 40.4 % | n.a. |
| C₁₄ mono-glucoside | 16.1 % | n.a. |
| C₁₆ mono-glucoside | n.a. | n.a. |

### 3. Formulation compatibility

To several commercial sunscreens containing titanium dioxide (nano) (SUN DANCE sensitive Sonnenbalsam SPF 30, SUN DANCE anti-age straffende Sonnenmilch SPF 30, SUN DANCE Sport Sonnen Light Lotion SPF 20, SUN DANCE Sonnen Light Lotion SPF 20 mit Kokosduft) Sun Ozon Sonnenspray SPF 30) either 10 wt.-% (i.e. 5 wt.-% active) of either Tinosorb^{®} M or the dispersion according to example 1 has been added.

Whereas the addition of Tinosorb^{®} M resulted in all of the above cases in an agglomeration i.e. a cottage cheese like effect of the respective sunscreen, the addition of the dispersion according to the present invention did not alter the optical appearance of the respective sunscreen.

As reference a sunscreen not containing titanium dioxide (nano) was tested (Coppertone Sport SPF 50) which resulted in both cases in no alteration of the optical appearance.

Furthermore, the formulations as outlined in table 2 have been prepared and analysed via microscopy as well as visually to assess the acceptability of the product form. As can be retrieved, only the samples prepared with a dispersion according to the present invention lead to an acceptable product form.

| **INCI *(Tradename)*** | **Ref 1** | **Inv 1** | **Ref 2** | **Inv 2** | **Ref 3** | **Inv 3** |
|---|---|---|---|---|---|---|
| Octocrylene *(Parsol^{®} 340)* | 10.00 | 10.00 | 10.00 | 10.00 | 10.00 | 10.00 |
| C12-15 Alkyl Benzoate *(Finsolv^{®} TN)* | 5.00 | 5.00 | 5.00 | 5.00 | 5.00 | 5.00 |
| Dicaprylyl Ether *(Cetiol*^{®} *OE)* | 2.00 | 2.00 | 2.00 | 2.00 | 2.00 | 2.00 |
| Homosalate *(Parsol^{®} HMS)* | 8.00 | 8.00 | 8.00 | 8.00 | 8.00 | 8.00 |
| Ethylhexyl Salicylate *(Parsol EHS)* | 5.00 | 5.00 | 5.00 | 5.00 | 5.00 | 5.00 |
| Titanium Dioxide (and) Silica *(Eusolex^{®} T-AVO)* | 4.50 | 4.50 | | | | |
| Titanium Dioxide (and) Aluminum Hydroxide (and) Stearic Acid *(Tayca MT-100TV)* | | | 4.50 | 4.50 | | |
| Titanium Dioxide (and) Silica (and) Dimethicone *(Parsol*^{®} *TX)* | | | | | 4.50 | 4.50 |
| Butyl Methoxydibenzoylmethane *(Parsol*^{®} *1789)* | 4.00 | 4.00 | 4.00 | 4.00 | 4.00 | 4.00 |
| VP/Hexadecene Copolymer (Antaron V-216) | 1.00 | 1.00 | 1.00 | 1.00 | 1.00 | 1.00 |
| Aqua *(WATER DEM)* | Ad 100 | Ad 100 | Ad 100 | Ad 100 | Ad 100 | Ad 100 |
| Glycerin | 6.00 | 6.00 | 6.00 | 6.00 | 6.00 | 6.00 |
| Carbomer *(Carbopol*^{®} *Ultrez 30)* | 0.10 | 0.10 | 0.10 | 0.10 | 0.10 | 0.10 |
| Xanthan Gum *(Keltrol*^{®} *CG-T)* | 0.15 | 0.15 | 0.15 | 0.15 | 0.15 | 0.15 |
| Acrylates/C10-30 Alkyl Acrylate Crosspolymer *(Pemulen*^{®} *TR-2)* | 0.30 | 0.30 | 0.30 | 0.30 | 0.30 | 0.30 |
| Sodium-EDTA *(Na-EDTA)* | 0.10 | 0.10 | 0.10 | 0.10 | 0.10 | 0.10 |
| Phenoxyethanol (and) Ethylhexylglycerin (Euxyl^{®} PE 9010) | 1.00 | 1.00 | 1.00 | 1.00 | 1.00 | 1.00 |
| Alcohol *(Ethanol)* | 8.00 | 8.00 | 8.00 | 8.00 | 8.00 | 8.00 |
| Methylene Bis-Benzotriazolyl Tetramethylbutylphenol (Tinosorb^{®} M) | 10.00 | | 10.00 | | 10.00 | |
| Methylene Bis-Benzotriazolyl Tetramethylbutylphenol dispersion (Example 1) | | 10.00 | | 10.00 | | 10.00 |
| pH | 6.16 | 6.23 | 6.53 | 6.41 | 6.01 | 6.07 |
| Agglomeration | strong | few | strong | none | strong | none |
| Phase separation | no | no | yes | no | no | no |
| Acceptable product form | No | Yes | No | Yes | No | Yes |

## Claims

1. An aqueous dispersion consisting essentially of
(i) 30-70 wt.-%, based on the total weight of the aqueous dispersion, of a nano-sized, organic, insoluble UV-absorber,
(ii) 2 to 15 wt.-%, based on the total weight of the aqueous dispersion, of a C₈₋₁₀alkyl poly-glucoside consisting essentially of caprylyl (C₈) and capryl (C₁₀) poly-glucoside,
(iii) 0 to 3 wt.-%, based on the total weight of the aqueous dispersion of at least one additive, and
(iv) 25 to 60 wt.-%, based on the total weight of the aqueous dispersion, of water,
wherein the term insoluble refers to an UV absorbers which exhibits a solubility at RT (i.e. ~ 22°C) in C₁₂₋₁₅ alkyl benzoate, propyleneglycol, mineral oil and in water of less than 0.01 wt.-% and the term nano-sized refers to a mean particle size distribution Dv50 determined by light scattering of less than 200 nm.

2. The aqueous dispersion according to claim 1, **characterized in that** the nano-sized, organic, insoluble UV-absorber is methylene bis-benzotriazolyl tetramethylbutylphenol or 2,4,6-tris(biphenyl-4-yl)-1,3,5-triazin, preferably methylene bis-benzotriazolyl tetramethylbutylphenol.

3. The aqueous dispersion according to claim 1 or 2, **characterized in that** the at least one additive is selected from the group consisting of wetting agents, thickeners and anti-foaming agents as well as mixtures thereof.

4. The aqueous dispersion according to claim 3, **characterized in that** the at least one additive is selected from the group consisting of propyleneglycol, a silicon anti-foam agent, xanthan gum and gellan gum as well as mixtures thereof.

5. The aqueous dispersion according to any one of claims 1 to 4, **characterized in that** the C₈₋₁₀ alkyl poly-glucoside has a ratio (%/%) of caprylyl (C₈) mono-glucoside to capryl (C₁₀) mono-glucoside in the range of 2:1 to 1:2.

6. A cosmetic composition comprising an aqueous dispersion according to anyone of claims 1 to 5 and at least one inorganic micropigment, **characterized in that** the cosmetic composition contains no more than 0.1 wt.-% of any C₁₂₋₁₆ alkyl poly-glucoside, based on the total weight of the cosmetic composition.

7. The cosmetic composition according to claim 6, **characterized in that** the amount (total) of the at least one inorganic micropigment is selected in the range of 0.1 to 40 wt.-%, based on the total weight of the cosmetic composition.

8. The cosmetic composition according to claim 6 or 7, **characterized in that** the amount of the aqueous dispersion in the cosmetic composition is selected in the range of 0.1 to 20 wt.-%, based on the total weight of the cosmetic composition.

9. The cosmetic composition according to any one of claims 6 to 8, **characterized in that** the inorganic micropigment is an inorganic UV filter or a coloring agent.

10. The cosmetic composition according to claim 9, **characterized in that** the inorganic micropigment is a titanium dioxide coated with at least one coating, preferably with aluminium hydroxide, a polyol, silica, a silicon oil or an alkyl silane.

11. The cosmetic composition according to claim 10, **characterized in that** the titanium dioxide is a double coated titanium dioxide having an inner aluminium hydroxide or inorganic silica coating and an outer organic coating selected from the group consisting of simethicone, methicone, dimethicone, polysilicone-15, cetyl phosphate, stearic acid and mixtures thereof.

12. The cosmetic composition according to claim 9, **characterized in that** the coloring agent is an iron oxide or a titanium dioxide having a particle size selected in the range of .001 to 150 µm.

13. The cosmetic composition according to any one of claims 6 to 12 **characterized in that** the cosmetic composition is an oil-in-water (O/W) emulsion comprising an oily phase dispersed in an aqueous phase.

14. The cosmetic composition according to any one of claims 6 to 13, **characterized in that** the composition is a sunscreen, or a make-up/ foundation composition.

## Patentansprüche

1. Wässrige Dispersion, im Wesentlichen bestehend aus
(i) 30-70 Gew.-%, bezogen auf das Gesamtgewicht der wässrigen Dispersion, an einem nanoskaligen organischen unlöslichen UV-Absorber,
(ii) 2 bis 15 Gew.-%, bezogen auf das Gesamtgewicht der wässrigen Dispersion, an einem C₈₋₁₀-Alkylpolyglucosid, das im Wesentlichen aus Caprylyl(C₈)- und Capryl(C₁₀)polyglucosid besteht,
(iii) 0 bis 3 Gew.-%, bezogen auf das Gesamtgewicht der wässrigen Dispersion, an wenigstens einem Zusatzstoff und
(iv) 25 bis 60 Gew.-%, bezogen auf das Gesamtgewicht der wässrigen Dispersion, Wasser,
wobei sich der Begriff "unlöslich" auf einen UV-Absorber bezieht, der eine Löslichkeit bei RT (d. h. ~22 °C) in C₁₂-₁₅-Alkylbenzoat, Propylenglycol, Mineralöl und in Wasser von weniger als 0,01 Gew.-% aufweist, und sich der Begriff "nanoskalig" auf eine mittlere Partikelgrößenverteilung Dv50, bestimmt durch Lichtstreuung, von weniger als 200 nm bezieht.

2. Wässrige Dispersion nach Anspruch 1, **dadurch gekennzeichnet, dass** der nanoskalige organische unlösliche UV-Absorber Methylen-bis-benzotriazolyltetramethylbutylphenol oder 2,4,6-Tris(biphenyl-4-yl)-1,3,5-triazin ist, vorzugsweise Methylen-bis-benzotriazolyltetramethylbutylphenol.

3. Wässrige Dispersion nach Anspruch 1 oder 2, **dadurch gekennzeichnet, dass** der wenigstens eine Zusatzstoff ausgewählt ist aus der Gruppe bestehend aus Netzmitteln, Verdickungsmitteln und Entschäumern sowie Gemischen davon.

4. Wässrige Dispersion nach Anspruch 3, **dadurch gekennzeichnet, dass** der wenigstens eine Zusatzstoff ausgewählt ist aus der Gruppe bestehend aus Propylenglycol, einem Silicium-Entschäumer, Xanthangummi und Gellangummi sowie Gemischen davon.

5. Wässrige Dispersion nach einem der Ansprüche 1 bis 4, **dadurch gekennzeichnet, dass** das C₈₋₁₀-Alkylpolyglucosid ein Verhältnis (%/%) von Caprylyl(C₈)monoglucosid zu Capryl(C₁₀)monoglucosid in dem Bereich von 2:1 bis 1:2 aufweist.

6. Kosmetische Zusammensetzung umfassend eine wässrige Dispersion nach einem der Ansprüche 1 bis 5 und wenigstens ein anorganisches Mikropigment, **dadurch gekennzeichnet, dass** die kosmetische Zusammensetzung nicht mehr als 0,1 Gew.-% an einem beliebigen C₁₂₋₁₆-Alkylpolyglucosid, bezogen auf das Gesamtgewicht der kosmetischen Zusammensetzung, enthält.

7. Kosmetische Zusammensetzung nach Anspruch 6, **dadurch gekennzeichnet, dass** die Menge (gesamt) des wenigstens einen anorganischen Mikropigments in dem Bereich von 0,1 bis 40 Gew.-%, bezogen auf das Gesamtgewicht der kosmetischen Zusammensetzung, ausgewählt ist.

8. Kosmetische Zusammensetzung nach Anspruch 6 oder 7, **dadurch gekennzeichnet, dass** die Menge der wässrigen Dispersion in der kosmetischen Zusammensetzung in dem Bereich von 0,1 bis 20 Gew.-%, bezogen auf das Gesamtgewicht der kosmetischen Zusammensetzung, ausgewählt ist.

9. Kosmetische Zusammensetzung nach einem der Ansprüche 6 bis 8, **dadurch gekennzeichnet, dass** das anorganische Mikropigment ein anorganischer UV-Filter oder ein Farbmittel ist.

10. Kosmetische Zusammensetzung nach Anspruch 9, **dadurch gekennzeichnet, dass** das anorganische Mikropigment ein Titandioxid ist, das mit wenigstens einer Beschichtung beschichtet ist, vorzugsweise mit Aluminiumhydroxid, einem Polyol, Siliciumdioxid, einem Silikonöl oder einem Alkylsilan.

11. Kosmetische Zusammensetzung nach Anspruch 10, **dadurch gekennzeichnet, dass** das Titandioxid ein doppelt beschichtetes Titandioxid mit einer inneren Aluminiumhydroxid- oder anorganischen Siliciumdioxidbeschichtung und einer äußeren organischen Beschichtung ausgewählt aus der Gruppe bestehend aus Simethicon, Methicon, Dimethicon, Polysilicon-15, Cetylphosphat, Stearinsäure und Gemischen davon ist.

12. Kosmetische Zusammensetzung nach Anspruch 9, **dadurch gekennzeichnet, dass** das Farbmittel ein Eisenoxid oder ein Titandioxid mit einer Partikelgröße ausgewählt aus dem Bereich von 0,001 bis 150 µm ist.

13. Kosmetische Zusammensetzung nach einem der Ansprüche 6 bis 12, **dadurch gekennzeichnet, dass** die kosmetische Zusammensetzung eine Öl-in-Wasser(O/W)-Emulsion ist, die eine in einer wässrigen Phase dispergierte ölige Phase umfasst.

14. Kosmetische Zusammensetzung nach einem der Ansprüche 6 bis 13, **dadurch gekennzeichnet, dass** die Zusammensetzung ein Sonnenschutzmittel oder eine Makeup/Grundlage-Zusammensetzung ist.

## Revendications

1. Dispersion aqueuse constituée essentiellement de
(i) 30-70 % en poids, par rapport au poids total de la dispersion aqueuse, d'un absorbeur d'UV organique insoluble de taille nanométrique,
(ii) de 2 à 15 % en poids, par rapport au poids total de la dispersion aqueuse, d'un alkyle en C₈₋₁₀-polyglucoside constitué essentiellement de caprylyl (C₈) et capryl (C₁₀) polyglucoside,
(iii) 0 à 3 % en poids, par rapport au poids total de la dispersion aqueuse, d'au moins un additif, et
(iv) 25 à 60 % en poids, par rapport au poids total de la dispersion aqueuse, d'eau,
dans laquelle le terme insoluble fait référence à un absorbeur d'UV qui présente une solubilité à TA (c.-à-d. ~ 22 °C) dans un benzoate d'alkyle en C₁₂₋₁₅, le propylèneglycol, une huile minérale et dans l'eau inférieure à 0,01 % en poids et le terme de taille nanométrique fait référence à une distribution granulométrique moyenne Dv50 déterminée par diffusion de la lumière inférieure à 200 nm.

2. Dispersion aqueuse selon la revendication 1, **caractérisée en ce que** l'absorbeur d'UV organique insoluble de taille nanométrique est méthylène bis-benzotriazolyl tétraméthylbutylphénol ou 2,4,6-tris(biphényl-4-yl)-1,3,5-triazine, de préférence méthylène bis-benzotriazolyl tétraméthylbutylphénol.

3. Dispersion aqueuse selon la revendication 1 ou 2, **caractérisée en ce que** l'au moins un additif est choisi dans le groupe constitué par les agents mouillants, les épaississants et les agents antimousse ainsi que leurs mélanges.

4. Dispersion aqueuse selon la revendication 3, **caractérisée en ce que** l'au moins un additif est choisi dans le groupe constitué par le propylèneglycol, un agent antimousse de silicone, la gomme xanthane et la gomme gellane ainsi que leurs mélanges.

5. Dispersion aqueuse selon l'une quelconque des revendications 1 à 4, **caractérisée en ce que** l'alkyle en C₈₋₁₀-polyglucoside présente un rapport (%/%) de caprylyl (C₈) mono-glucoside sur capryl (C₁₀) mono-glucoside dans la plage de 2:1 à 1:2.

6. Composition cosmétique comprenant une dispersion aqueuse selon l'une quelconque des revendications 1 à 5 et au moins un micropigment inorganique, **caractérisée en ce que** la composition cosmétique ne contient pas plus de 0,1 % en poids d'un quelconque alkyle en C₁₂₋₁₆-polyglucoside, par rapport au poids total de la composition cosmétique.

7. Composition cosmétique selon la revendication 6, **caractérisée en ce que** la quantité (totale) de l'au moins un micropigment inorganique est choisie dans la plage de 0,1 à 40 % en poids, par rapport au poids total de la composition cosmétique.

8. Composition cosmétique selon la revendication 6 ou 7, **caractérisée en ce que** la quantité de la dispersion aqueuse dans la composition cosmétique est choisie dans la plage de 0,1 à 20 % en poids, par rapport au poids total de la composition cosmétique.

9. Composition cosmétique selon l'une quelconque des revendications 6 à 8, **caractérisée en ce que** le micropigment inorganique est un filtre UV inorganique ou un agent colorant.

10. Composition cosmétique selon la revendication 9, **caractérisée en ce que** le micropigment inorganique est un dioxyde de titane revêtu par au moins un revêtement, de préférence par de l'hydroxyde d'aluminium, un polyol, une silice, une huile de silicium ou un alkylsilane.

11. Composition cosmétique selon la revendication 10, **caractérisée en ce que** le dioxyde de titane est un dioxyde de titane à double revêtement ayant un revêtement interne d'hydroxyde d'aluminium ou de silice inorganique et un revêtement externe organique choisi dans le groupe constitué par la siméthicone, la méthicone, la diméthicone, la polysilicone-15, le phosphate de cétyle, l'acide stéarique et leurs mélanges.

12. Composition cosmétique selon la revendication 9, **caractérisée en ce que** l'agent colorant est un oxyde de fer ou un dioxyde de titane ayant une taille de particule choisie dans la plage de 0,001 à 150 µm.

13. Composition cosmétique selon l'une quelconque des revendications 6 à 12, **caractérisée en ce que** la composition cosmétique est une émulsion huile-dans-eau (H/E) comprenant une phase huileuse dispersée dans une phase aqueuse.

14. Composition cosmétique selon l'une quelconque des revendications 6 à 13, **caractérisée en ce que** la composition est un écran solaire, ou une composition de maquillage/fond de teint.
